Europäisches Patentamt

European Patent Office

Office européen des brevets

(19)

(11) EP 1 318 472 A2

(12) EUROPEAN PATENT APPLICATION

(43) Date of publication:
11.06.2003 Bulletin 2003/24

(51) Int Cl.7: G06F 19/00

(21) Application number: 02027262.1

(22) Date of filing: 06.12.2002

(84) Designated Contracting States:
AT BE BG CH CY CZ DE DK EE ES FI FR GB GR
IE IT LI LU MC NL PT SE SI SK TR
Designated Extension States:
AL LT LV MK RO

(30) Priority: 10.12.2001 US 337150 P

(71) Applicant: FRAUNHOFER-GESELLSCHAFT ZUR
FÖRDERUNG DER
ANGEWANDTEN FORSCHUNG E.V.
80636 München (DE)

(72) Inventors:
• Zien, Alexander
  53121 Bonn (DE)
• Hanisch, Daniel
  53129 Bonn (DE)

(74) Representative:
Meyers, Hans-Wilhelm, Dr.Dipl.-Chem. et al
Patentanwälte
von Kreisler-Selting-Werner
Postfach 10 22 41
50462 Köln (DE)

(54) **Method for the analysis of molecular expression data and biological networks**

(57)    A method for the joint analysis of molecular expression data and biological networks by clustering comprising the steps of

-   defining a matrix of distances between molecules or sets of molecules that incorporate both the relation of corresponding expression profiles and information on their relation within a biological network; and

-   clustering the molecules based on said distances.

Figure 2

## Description

### Background and State of the Art

Most methods, clustering and others, for the analysis of gene expression data are also applicable to other types of molecular expression data, e.g. protein expression data or concentrations of small molecules. Without loss of generality, in the following discussion it will be focused on gene expression data simply because it is at present the most easily available data type and because the vast majority of publications and applications deal with them.

### Clustering of Gene Expression Data

*Clustering* is a general unsupervised learning technique that is applied in many areas. It is also one of the earliest and most popular approaches to the analysis of gene expression data. Since gene expression data can be arranged in the form of a matrix, with rows representing genes and columns representing samples, there are two different criteria by which clustering can be done. One will focus here on the clustering of genes.

Many established methods for clustering were applied to gene expression data. They can be roughly divided into

- hierarchical;

- self organizing maps (SOMs); and

- centroidal methods (e.g., k-means).

In addition, new clustering methods were designed that are supposed to be particularly appropriate for gene expression data. These include

- gene shaving [6]; and

- CLICK [12].

There are also methods that simultaneously cluster genes and samples and thereby aim to improve the results [1].

Being a method of unsupervised machine learning, pure clustering methods do not take into account available knowledge on the problem domain. However, such knowledge is available, often in the form of graphs of interactions, as described below.

### Biological Networks

It is commonly assumed that gene expression data contains valuable information about biological networks. *Biological networks* relate genes, gene products or groups of those (e.g., protein complexes or protein families) to each other in the form of a graph. Such biological networks are a concise and useful representation of available knowledge on biochemical interactions of important molecules in cells. For example, metabolic networks can be seen as Petri nets (bipartite graphs), where metabolites are inputs or outputs of reactions, which are catalyzed by enzymes. Other examples are regulatory networks, where proteins interact directly in order to confer signals or influence the expression of other genes (as, e.g., in TRANSPATH) and networks of interacting proteins, which contain experimentally determined protein-protein interactions (e.g. DIP).

*Genetic network reconstruction* is the attempt to compute such networks from scratch using measured gene expression data. The resulting networks may be Boolean, linear, or Bayesian models, for example. Network reconstruction can also be based on clusters of genes and thereby yield coarse-grain genetic networks.

### Joint Analysis of Gene Expression Data and Biological Networks

Several approaches have been developed that relate gene expression data to the topology of biological networks. In [9], gene expression data is visualized on top of a metabolic network. In [15], putative pathways are generated from the biochemical network and then scored according to the gene expression data in order to distinguish pathways that are realized and active in the measured samples. In [4] and [14], genes are first clustered according to the expression data and then mapped onto networks. In [13], a core of a pathway can be expanded by genes according to a scoring function that is computed from gene expression data. In [5], a network topology is selected among a given set of putative alternatives using Bayesian reasoning. In [7], a network is generated from co-occurences of gene names in literature abstracts. Then, subnets (called clusters in that paper) are extracted from that network and subsequently scored with respect to gene expression data. This approach is similar in spirit to the pathway scoring described in [15]. In [10], the authors describe a method for the concurrent clustering of two graphs, exemplified for biological networks and genomes. They envision the use for other types of data, including the application to biological networks and networks computed from gene expression data, but no details are described.

In [11], the authors show a method to incorporate transcription factor binding site information into the clustering of gene expression data. However, they do not consider the incorporation of knowledge on biological networks, and it is not obvious how to do so using their framework.

**Brief Description of the Invention**

Substantial efforts have been made to develop rather indirect ways to make use of networks for the analysis of gene expression data, as discussed above. However, those methods that are developed to incorporate other data types into gene expression analysis more directly (e.g., [11]) work well for almost all other data types (including phylogenetic profiles, transcription factor binding sites, etc.), but cannot readily make use of adjacency relations in biological networks.

An object of the invention is to provide a new method for the integration of gene expression data with knowledge on biological (protein/gene) networks. Basically, this is accomplished by incorporating knowledge on the network structure into the distance matrix that is used for clustering apart from the gene expression data.

The invention provides a method for the joint analysis of molecular expression data and biological networks by clustering comprising the steps of

- defining a matrix of distances between molecules or sets of molecules that incorporate both the relation of corresponding expression profiles and information on their relation within a biological network; and

- clustering the molecules based on said distances.

In an embodiment of the invention the distance between two molecules is defined by combining a distance function on the corresponding expression data with a distance function on the biological network, such that the resulting distance of two molecules is monotonically increasing with increasing expression data distance for constant biological network distance, and monotonically increasing with increasing biological network distance for constant expression data distance.

Preferably the distance between two molecules is defined as infinite (or prohibitively high) if

- said molecules are not adjacent in a given biological network;

- said molecules are nodes in a given biological network but are not adjacent to each other;

- said molecules are not closer to each other (in terms of path length) than a given threshold in a given biological network; or

- said molecules are more distant to each other (in terms of path length) than a given threshold in a given biological network;

and where the distance is calculated from the expression data alone, otherwise.

In another embodiment the distance between two molecules is defined as infinite (or prohibitively high) if the corresponding expression distance exceeds a given threshold, and where the distance is calculated from the biological network alone otherwise.

Furthermore, the distance between two molecules is defined by combining a score deduced from the expression data with a distance function deduced from the biological network, where the biological network distance may be either continuous or discrete. Preferably, the score of a molecule reflects the degree of differential expression between two states or types of cells.

In a further embodiment of the invention the distance between two molecules is defined by combining a score deduced from the biological network with a distance function deduced from the expression data, where the expression distance may be either continuous or discrete.

According to the invention the clustering algorithm is in particular

- an average linkage clustering algorithm;

- a hierarchical clustering algorithm;

- k-means clustering;

- self-organizing networks (SOMs); or

- a centroidal clustering algorithm

- a hypergraph clustering algorithm.

In particular according to the invention the molecular expression data is

- gene expression data;

- protein expression data; or

- metabolite abundances.

**Detailed description of the invention**

The goal of the clustering, as is subject of the invention, is to yield clusters that form biologically reasonable groups of genes, e.g. subnets (so-called pathways) of the biological network. In order to achieve this goal, the distances are defined to be smaller between genes that show a close relationship in the network than between genes that are less closely related in the network. The resulting clusters will then tend to be more similar to subnetworks of the given biological network than with a conventional distance function that is based on gene expression data only. Attributes of the edges (e.g., activating or inhibiting relationship) can be used in the definition of the distance function. Furthermore, it is possible to also incorporate other features of the nodes (apart from expression data), e.g. subcellular localization, into the distance function.

**Examples of Embodiments**

In a preferred embodiment of this invention, it is used to locate areas of interest in a large network relating to a two class comparison of cell samples. Here, the two classes can be, e.g., healthy and diseased tissue from patients, or cultivated tissue that is treated with a drug or stimulant or untreated, respectively. From the gene expression data of several samples of both classes, genes can be identified that are differentially expressed in both states. Such studies are frequently performed by the pharmaceutical industry and by others, since they can yield valuable insights into the molecular causes of diseases or into the molecular details of drug action. The biochemical network that is used in this preferred embodiment may consist not only of proved facts, but also of putative interactions or hypotheses. For example, interactions may be incorporated into the network which were shown to take place in different cell types or even different organisms than those that the samples relate to. Such networks can easily become very large, and a method that guides human experts to areas of interest in such networks is highly desirable. To do so, a distance function can be composed of two parts as follows. The first part is the distance according to the graph, e.g. the minimal path length which can be efficiently computed by common graph algorithms (e.g. the Floyd-Warshall algorithm). The second part is built on the degrees of differential expression that are measured and computed for the nodes and assign high distances to nodes that do not both show good evidence for differential regulation. In addition, the distance function may take into account the connectedness of the nodes (i.e., genes or their protein products) and by increasing the edge length of highly connected nodes avoid the unwanted linking of separate pathways that, are only connected by a few hubs (i.e., highly connected but rather unspecific nodes). In addition, the distance function can take the origin of and confidence in an interaction into account by considering partners of proved interactions to be less distant than those of putative or hypothesized interactions.

In a second preferred embodiment of this invention, it is used with a similar intention as the pathway scoring described in [15]. Here, the goal is to identify subgraphs that are realized in all or some of the investigated samples. In contrast, most subgraphs of a network that is created by merging interactions from different organisms and cell types can be expected to not be realized under any condition in the investigated cell types. In order to find realized pathways, the distance function should reward both short connecting paths in the biological graph and related expression profiles with small distance values.

In another preferred embodiment of this invention, clusters are desired that correspond well to groups of genes with similar functions.

**Generalizations of Distance Functions**

Conventionally, a distance function yields a real value for every pair of objects reflecting their degree of relatedness. Obviously, similarity functions can be used for the construction of equivalent distance functions by inverting their values. Also these two generalizations of distance functions are considered to be in the context of this invention.

First, if one is interested in finding few clusters with certain distinguished genes and do not care about the other genes, one can use a scoring function instead of a distance function, thus associating a real value with every single object. For example, in the first preferred embodiment, one can combine a biological graph distance function (based on adjacency) with a score for differential regulation derived from the gene expression data. One will then find clusters of connected differentially expressed genes. The scoring function need not have any mathematical properties of a distance function.

Second, for most clustering algorithms it is required to be able to calculate the distance between two sets of objects. For example, hierarchical linkage clustering starts with a separate cluster for each object and then iteratively merges clusters that are close to each other. In k-means clustering, the distance to a virtual cluster centroid can be expressed as the distance to the set of objects that belong to the cluster. A common approach is to compute the average of all pair wise distances of elementary objects from both clusters. This does not make sense for certain kinds of biological network distances where some distance values are taken to be infinite. There, it is more reasonable to, e. g., consider two clusters of genes as adjacent (resulting in a finite distance) if there exists a pair of nodes from both sets that is adjacent in the graph. As a second example, if one uses gene expression scores instead of distances, there may be more appropriate methods to combine scores than averaging. For scores that are probabilities (e.g., of differential expression), one can use meta-analysis methods (e.g. Fisher's Chi square method) to combine them.

Finally, the distance function can be extended to be defined on higher triples of objects instead of just pairs. This can be especially useful for distances on graphs, as demonstrated in the following two examples. If the resulting clusters are required to be connected subgraphs, an infinite distance can be assigned to every pair of objects or clusters of objects that are not adjacent. This can lead to very efficient implementations of most clustering algorithms, e.g. of the linkage clustering, since not all pairs of objects need to be considered for merging but only adjacent ones. If, however, two related objects (e.g., two genes that show very similar gene expression profiles) are separated in the graph by a third node with dissimilar behaviour, they may not be clustered although this may be desirable. If triples of objects are considered, they may be merged in such a case. This extension leads to the use of hypergraph clustering techniques (e.g. HMetis).

**Embodiment of the invention**

The focus of this embodiment is on the analysis of *co-regulated metabolic pathways* supported by gene expression measurements. Metabolic reactions are an integral part of every organism, and constitute fundamental cellular processes such as protein synthesis or energy production. This setting is well suited to assess the feasibility of the approach of the invention as sets of metabolic reactions and associated pathways (e.g. KEGG) and gene expression data known to represent metabolic changes (e.g. [2]) are readily available and reasonably well understood.

**Metabolic network acquisition and representation**

In the setting of the invention metabolic networks consist of a set of chemical reactions, most of which are catalyzed by enzymes. Enzymatic reactions which can be assembled into a metabolic network are available in several databases. The KEGG database is chosen as it provides an organization of reactions into pathways as well as a large dataset of curated metabolic reactions. The pathways reflect commonly used categories and can be visualized on manually drawn pathway maps. Since the co-clustering procedure of this invention does not utilize this information, it is well suited as an independent evaluation guide. As of December 2001 the KEGG dataset consists of approximately 4000 metabolic reactions. Each reaction is realized in some organism and is annotated with a reaction identifier, functionally important educts and products, and the classification of the catalyzing enzyme. Each reaction may be uni- or bidirectional. One takes the combination of identifier and EC classifier to be a unique identifier for a metabolic reaction in the network. One assembles this set of reactions into a network in the form of a PETRI net, similar to the method described by Kueffner et al. [8]. The constructed PETRI net is essentially a bipartite graph in which one set of nodes (termed places) represents molecules (metabolites and enzymes) and the other set of nodes (called transitions) defines chemical reactions among the molecules. A directed edge between a molecule and a reaction implies that the molecule is product or educt of the reaction depending on the direction of the edge. Note that a catalyzing enzyme is educt and product simultaneously, i.e. two edges connect it to an associated transition. Enzyme identifier can occur multiple times as each may be able to catalyze multiple reactions. A unification of these nodes would introduce unwanted shortcuts into the metabolic network which may have no biological meaning. In fact, these nodes may be assigned to different clusters in the co-clustering procedure of the invention. This co-clustering method should be able to extract biologically plausible sub-networks in the light of the given gene expression data. This should partly correspond but not be restricted to defined KEGG pathways. Moreover, a network is constructed which is *not* specific to any single organism. Indeed, it would be possible to construct specialized networks for certain organisms based on the KEGG data. Such a network would consist of fewer reactions than the generic one as enzymes might not be present or unknown in the chosen organism. Therefore, it might exhibit a less connected and more cluster-like structure. This embodiment of the invention, however, reports detailed results only on the generic network. Thereby, the situation is emulated in organisms with little prior knowledge. In such organisms known reactions may be connected by generic ones to yield a hypothetical, but plausible network.

**Network distance function**

The underlying assumption for the network distance function of the invention is that enzymes are related according to their proximity in the network. This is reasonable, as the biological processes under consideration consist of successive metabolic reaction steps which constitute the network of the invention. In order to construct a distance function for the metabolic network $\delta_{net}$, one interprets the derived PETRI net as an undirected graph $G = (V, E)$ with node set $V$ and edge set $E$. In this case, $V$ is the set of all molecules, i.e. proteins and metabolites, and reactions of the network. Furthermore, a weight function is defined $w : E \to R$ which associates a weight with each edge. Let $W \subset V$ be the set of molecules for which gene expression data are available. Then one defines $\delta_{net}$: $W \times W \to R$ for two vertices $w_i$ $W_j \in W$ as the minimum weight of all paths connecting vertices $w_i$ and $w_j$. These minimal weights can be computed efficiently using basic graph algorithms. One first eliminates all $|V| - |W|$ superfluous vertices by considering for each such vertex x all pairs of neighbors. Let $(v_i, v_j)$ be such a pair of neighbouring vertices. If the weight $w(p_x)$ of the path $p_x = (v_i, x, v_j)$ is smaller than the weight of the path $p_v = (v_i, v_j)$, one connects $v_i$ and $v_j$ with an edge with weight $w(p_x)$. If all pairs of neighbors have been considered, the node x and all incident edges can be deleted. For the remaining $|W|$ nodes of interest one uses the Floyd-Warshall algorithm to compute all shortest paths. For dense graphs, the first step can be time-consuming as $|V|^2$ pairs of vertices have to be considered in the worst case, thus resulting in a worst case running time of $O(|V - W||V|^2)$. In the sparse network, however, this worst case running time is virtually never observed. When nodes are processed in order of increasing degree, it will often suffice to consider only a small fraction of $V$ as most nodes have few incident edges. The subsequent Floyd-Warshall algorithm takes time $O(|W|^3)$. Another option to compute the minimal weights is the Dijkstra shortest-path algorithm. This algorithm can compute the shortest path weights of one vertex to all other vertices in asymptotic running time $O(|V| \log|V| + |E|)$ for non-negative weight functions. As one needs to compute the weights for all vertices in the set of interest $W$, the overall running time to compute the network distance function is $O(|W||V| \log|V| + |W||E|)$. It was found that the first strategy is much faster for the networks under consideration. Reasons may be that the worst-case bound of the first strategy is not sharp for the class of graphs and the constant hidden in the $O$-notation is small. As the number of interesting vertices is usually smaller than the number of all vertices and the network under consideration is sparse, computation of this distance function is feasible for even large networks. Furthermore, in the procedure of the invention graph distances only need to be computed once for each network. The simplest plausible choice for the weight function is the uniform weighting $w(e) = 1$ for all $e \in E$. The resulting distance function is termed $\delta_{uni,net}$. Recently, it has been suggested that metabolic networks exhibit a scale-free structure. Indeed, this is true for the network constructed from KEGG reactions. One characteristic of networks with a scale-free structure is that relatively few high-degree nodes are found. These so-called hubs often constitute unspecific or ubiquitous molecules (e.g. ATP or NH3). Thus, the connections introduced by such nodes may be biologically unimportant or even misleading. In other words, one prefers clusters in which all genes are reachable from each other in a few steps without hubs. This can be reflected in the distance function using the degree of a vertex $v$, i.e. the number of edges incident to $v$, termed $deg(v)$. The alternative weighting function for an edge $e$ between two vertices $v$ and $w$ is defined as

$$w(e) = \begin{cases} deg(v) & \text{if } v \text{ is a molecule} \\ deg(w) & \text{otherwise.} \end{cases}$$

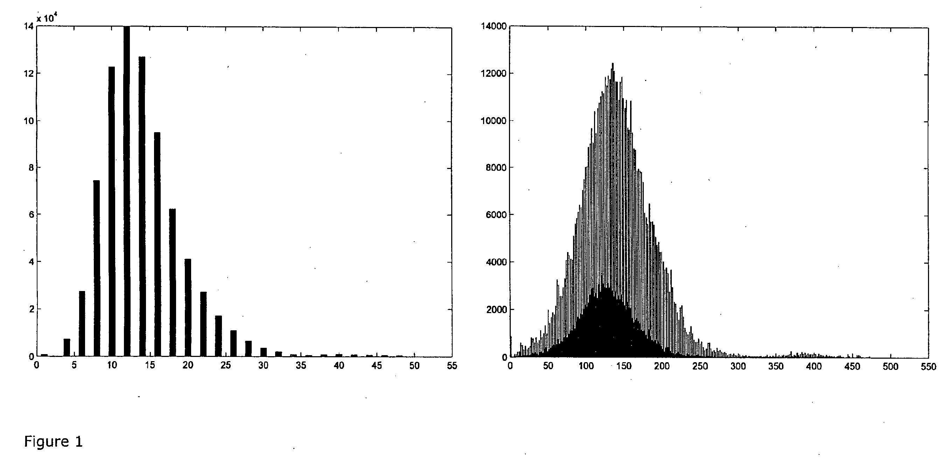

Note that the graph is bipartite, i.e. every edge connects one molecule with one reaction. One does not use the sum $deg(v) + deg(w)$ as one does not want to penalize reactions with many involved molecules, but only substrates participating in different biological processes. The resulting distance function is termed $\delta_{norm, net}$. Histograms of the distance distributions for the metabolic network according to both functions are depicted in Figure 1.

Fig. 1 shows on the left, histogram of network distances without degree normalization and on the right, histogram of network distances with degree normalization. In both graphs a fraction of 0.9% of the distances was infinite. These are omitted in the plots.

Several alternative distance functions for gene expression measurements have been proposed, while the most popular choice in the case of time-series data is the Pearson correlation coefficient, as suggested by Eisen et al. [3]. This coefficient quantifies the degree of linear dependence between two time-courses of gene expression levels. In this embodiment of the invention, log-ratio transformed data are used, i.e. for each sample of interest the logarithm of the ratio of the sample and a control measurement is computed. If $G_{ik}$ represents this value for gene $g_i$ at time point $k$, then the correlation coefficient $\rho$ is defined as

$$\rho(g_i, g_j) = \frac{1}{N} \sum_k \left( \frac{G_{ik} - \mu_i}{\sigma_i} \right) \left( \frac{G_{jk} - \mu_j}{\sigma_j} \right),$$

where $\mu_i$ and $\sigma_i$ denote mean and standard deviation of the transformed time series data of gene $i$. The correlation coefficient can be easily converted into a distance measure in the range 0 to 2 by

$$\delta_{exp} (g_i, g_j) = 1 - \rho (g_i, g_j).$$

This distance function quantifies the degree of dissimilarity for the gene expression data set. Here one considers anti-correlated genes as most distant. The use of correlation as a distance function is reasonable in the setting of the invention as one expects a similar expression pattern between genes in successive (or related) reaction steps in metabolic pathways. However, one cannot expect to see perfect correlation of expression in a pathway for two reasons. First, gene expression measurements reflect the amount of mRNA in the sample, and thus, the amount of enzyme to be produced in the near future. Second, expression measurements are noisy with current high-throughput technology. Nevertheless, a coordinated change in the expression patterns of participating genes is to be expected when a metabolic pathway changes its activity level.

### Co-Clustering

In the following it is defined define how to combine the distance functions for networks and gene expression data and how to compute the desired clustering. Note that a large part of the following discussion applies not only to metabolic networks but also to more general biological networks.

### Combining nodes and genes

The network distance function $\delta_{net}$ operates on pairs of enzyme nodes in the graph, whereas the expression distance function $\delta_{exp}$ operates on pairwise expression measurements, i.e. genes of an organism. To construct a combined function, a mapping M that relates genes to enzyme nodes in the graph is required. For yeast, such a mapping is available from the MIPS database. In this database, E.C.-classifications are assigned to all ORFs with known metabolic function. This mapping is *not* one-to-one. Indeed, one ORF may have several enzymatic functions, and conversely several ORFs may map to one E.C. entry. In addition, each single EC number may correspond to several nodes in the network for reasons given above. To cope with this situation, the combined distance function is defined on the product set of genes and relevant nodes in the network. Members of this product set are termed *objects.* Thus, if *G* is the set of genes and *V* is the set of nodes in the network, then the mapping M $\subset$ *G*$\times$*V* defines the domain of the combined distance function and, thus, the set of objects used in the clustering procedure. To illustrate this, consider nodes with enzyme classification *Hexokinase* (EC number 2.7.1.1). There are two yeast proteins which are associated with this function, *HXK1* and *HXK2.* However, during diauxic shift, the expression of these two genes are strongly anticorrelated [2]. Conversely, the hexokinase belongs to the group of phosphotranferases. For instance, it can catalyze the conversion of alpha-D-glucose to alpha-D-glucose 6-phosphate (KEGG reaction ID *R*01786) as well as the conversion of D-glucosamine to D-glucosamine 6-phosphate (KEGG reaction ID *R01961*). While these reactions are similar, it is not clear a-priori that they are embedded in the same functional context and should therefore share a cluster. Indeed, in terms of the KEGG pathways these reactions occur in the glycolysis and aminosugars metabolism pathways, respectively. Consequently, the method of the invention constructs distinct objects for each gene/reaction pair. For example, the protein HXK1 would map to two distinct objects, i.e. $o_1 = (g_1, v_1) = $ (HXK1, EC 2.7.1.1/R01786) and $o_2 = (g_1, v_2) = $ (HXK1, EC 2.7.1.1/R01961). The protein HXK2 is treated analogously. The co-clustering results in a clustering of such objects which can be reduced to a clustering of vertices or genes as necessary.

## Combining distances

The combined distance function should assign a small distance to pairs of objects which are close in the network *and* show similar expression patterns. Objects which are far apart in the network and thus are presumably used in different biological context should be far apart according to the combined function. The same holds true for objects which are not co-regulated or even oppositely regulated, as one wants to extract co-regulated pathways. The largest distance should be assigned to objects which are far apart according to *both* distance measures. In addition, the combined function should be robust against noise in the data. For example, perfect correlation should not result in extraordinarily small distance in comparison with a good or moderate correlation as such differences might often be due to measurement noise. This in turn could lead to artifacts in the clustering procedure. Analogously, erroneously missing links between enzymes of interest in the network should not lead to prohibitively high distances. This robustness can be achieved by saturation at the extremes of the parameter space.

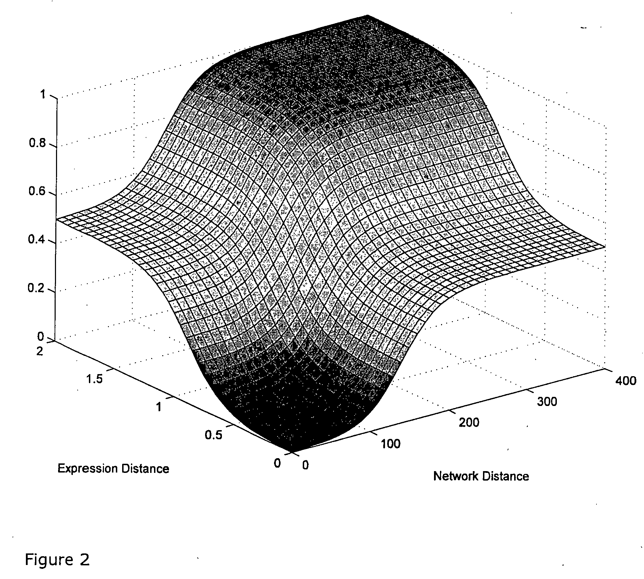

Fig. 2 shows a graph of combined distance function $\Delta_{norm}$.

One function capable of combining the individual distance functions into a joint one with the above properties is the sum of two logistic curves. This sigmoidal function attains its maximum for minimal x- and y-values and gradually declines with increasing x- and y-values. As the distance function of the invention needs to assign minimal values for minimal parameter values, the functional form of the combined function $\Delta$ for two objects $o_i, o_j \in M$ with corresponding genes $g_i, g_j$ and corresponding enzyme nodes in the graph $v_i, v_j$ is

$$\Delta(o_i, o_j) = 1\text{-}0.5 \times (\lambda_{exp}(g_i, g_j) + \lambda net(v_i, v_j)),$$

where

$$\lambda_\Psi(x_i, x_j) = \frac{1}{1 + e^{-s_\Psi(\delta_\Psi(x_i, x_j) - \nu_\Psi)}}$$

for $\psi \in \{exp, net\}$. The parameters $v_\psi, s_\psi \in R$ control the shape of the logistic curve. Essentially, a one-dimensional logistic curve is a smooth threshold function with value 1/2 at point $v_\psi$. The parameter $s_\psi$ controls the slope of the curve. One sets the parameter $v_\psi$ to the mean of the distance distributions of the network and expression distances, respectively. The parameter $s_\psi$ is chosen heuristically to yield a moderate slope ($s_\psi = 6/v_\psi$). The resulting combined distance function is shown in Figure 2. A validation of this combination can be found in the Results section. As pointed out above, genes and vertices may occur multiply in distinct objects. It needs to be considered whether pairs of these objects are assigned a sensible distance. The first case is that one vertex is assigned to several genes, i.e. more than one gene may fulfil the desired function or even all genes are needed to fulfil the enzymatic activity (e.g. as subunits in a complex). Then, one leaves the network distance of zero as implied by the distance function $\delta_{net}$ unchanged. This means that these objects share a cluster if their expression profiles do not disagree strongly. In the second case, when one gene is mapped to several vertices in the network, one sets the expression distance to the mean of all expression distances, i.e. to a normalized value of 1/2. The rationale for this is that distinct vertices in the network catalyze different reactions, and thus a single gene may play a role in different biological contexts. The perfect correlation of the expression measurements, however, might prevent the assignment to different clusters.

## Clustering methodology

From the large number of available cluster methods, one chooses a version of *hierarchical average linkage clustering.* Since the influential work of [3], this is one of the most popular clustering techniques for the analysis of gene expression measurements. Starting from the set of objects as singleton clusters, the method successively joins clusters with smallest average pair wise distance. The result of a hierarchical clustering procedure is a binary tree (or a dendrogram) in which each inner node represents a joining step of the procedure. To produce a set of clusters, the tree is cut by removing all nodes after a chosen joining step. One major problem arising in cluster analysis is to determine the appropriate number of clusters and thus the cutting point for the dendrogram. Various statistical measures exist in this regard. The *silhouette-coefficient* has been selected for the purposes of the invention. The coefficient measures the quality of a clustering by a comparison of the tightness and separation of clusters. Let *i* be any object in the clustering and A its corresponding cluster. Then

$$a(i) = \frac{1}{|A|-1} \sum_{j \in A, j \neq i} \Delta(i,j)$$

measures the average distance of *i* to all other objects in the cluster *A*. Then, compute for each cluster *C≠A*

$$d(i,C) = \frac{1}{|C|} \sum_{j \in C} \Delta(i,j)$$

to quantify the distance to other clusters. The minimum value

$$b(i) = \min_{C \neq A} d(i,C)$$

gives the distance of *i* to the second-best cluster. The silhouette value *s(i)* of *i* is then defined as

$$s(i) = \frac{b(i)-a(i)}{max\{a(i),b(i)\}}.$$

This value in the range [-1;1] quantifies how well object *i* fits into cluster *A*. If the *s(i)* is smaller than zero, the nearest cluster would be a better choice. The *average silhouette value* is the average of the *s(i)* over all objects in the clustering and is a measure for the quality of the clustering. It has to be noted that other measures may be important for the assessment of cluster quality as well. For example, one is interested in clusters of a certain size or compactness, which is not directly measured by the average silhouette coefficient. Nonetheless, it provides a good aid in choosing reasonable cutting points. One will plot this average silhouette value for different cut points of the dendrogram to find sensible clusterings.

**Results**

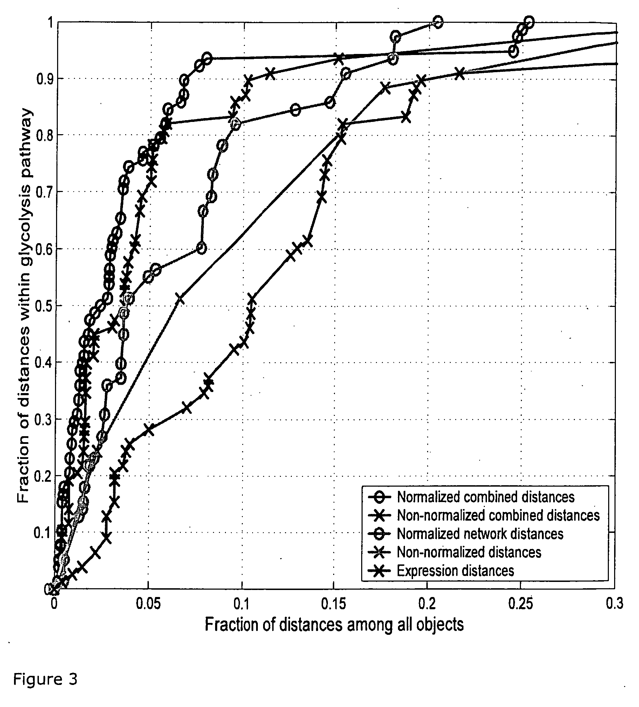

To evaluate the performance of the method of the invention, one uses the gene expression time series measurements conducted by DeRisi et al. [2] for the organism *S. cerevisiae* (yeast). In this data set, measurements for seven different time points are taken. In this experiment, yeast is inoculated into a sugar-rich medium. When the sugar is progressively depleted, the metabolism of yeast switches from anaerobic growth to aerobic respiration. This so-called diauxic shift involves changes in several metabolic processes which should be detectable by the method of the invention. DeRisi and co-workers manually analyze several pathways related to the diauxic shift which can serve as a standard of truth for the validation of the method of the invention. Of the 6101 yeast ORFs measured in these experiments, 642 have known metabolic functions according to the MIPS database. 884 nodes in the metabolic network derived from the KEGG database correspond to these enzymatic functions. Due to the multiplicity of the mapping, one arrives at 1571 objects to be clustered. In a first step, one compare the relative quality of the five defined distance functions, i.e. the gene expression distance function $\delta_{exp}$, the normalized and non-normalized network distance functions $\delta_{uni,net}$ and $\delta_{norm,net}$, and the two combined distance functions $\Delta_{uni}$ and $\Delta_{norm}$. DeRisi et al. found that the glycolysis pathway is significantly influenced by the diauxic shift. From this pathway ORFs are selected (and corresponding objects) which score highest in the pathway scoring method [15], i.e. show high co-regulation and constitute a complete reaction chain (YGL253W, YBR196C, YMR205C, YKL060C, YJR009C, YDR050C, YCR012W, YHR174W, YAL038W). As a figure of merit for the quality of a distance function, plot the fraction of distances among *all* objects against the fraction of pairwise distances of glycolysis objects found among these. In other words, investigate which fraction of relationships within the glycolysis pathway is already considered when one inspects a certain fraction of all distances. The resulting graph is found in Figure 3.

Fig. 3 shows the relative quality of defined distance functions. The higher the curve, the better the respective distance function discriminates related from unrelated objects.

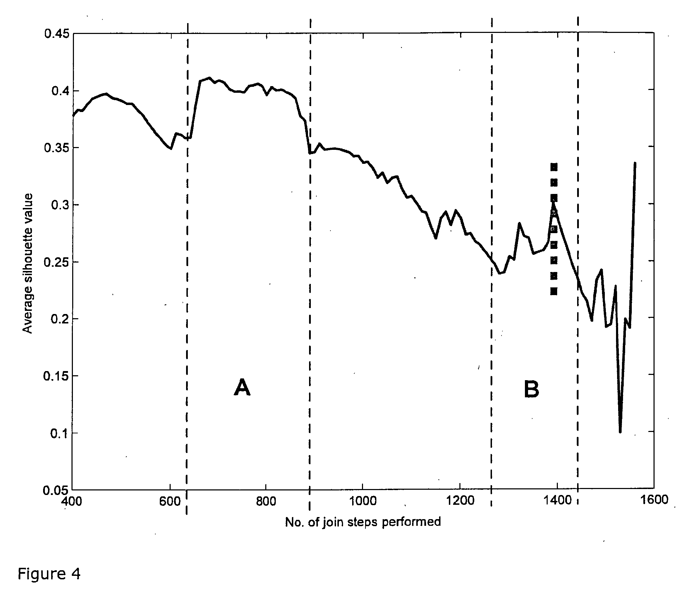

Although each distance function is far better than random, the combined distance functions clearly perform best. For instance, to consider 90 % of distances among glycolysis objects, one only needs to inspect approximately 6 % of the overall data, in contrast to 23 % for the expression distance function. Note that the expression distance function $\delta_{exp}$ begins with a moderate slope indicating that a large number of co-regulated objects is present. By utilizing network information, pairs of objects which are co-regulated but far apart in the network are not considered at an early stage. The steep slope of the combined functions shows that this is successfully accomplished. In addition, the normalized functions perform better than their non-normalized counterparts indicating that the scale-free structure of metabolic networks can provide additional distance information compatible with the definition of the glycolysis pathway and probably with the notion of metabolic pathways in general. In the following discussion, one focuses on the normalized combined distance function. As already hinted at by the above evaluation, the normalized distance function leads to biologically more plausible clusters. Especially, linking of clusters via ubiquitous nodes, such as $NH_3$, $CO_2$ or ATP, does not occur. After construction of the dendrogram based on $\Delta_{norm}$, the average silhouette value for a range of possible cutting points (cf. Figure 4) was computed to find a clustering reflecting the desired output, i.e. clusters corresponding to pathways with coordinated change in gene expression.

Fig.4 shows the average silhouette values for clusterings resulting from different cut-off values. The normalized network distance function is used. Cutting point is marked with dashed line.

Part A of Figure 4 has highest average silhouette values. Cutting points in this interval result in clusters of objects with multiple representations, i.e. objects sharing the same E.C. number and similar expression patterns or one gene mapped to several closely related nodes in the network. For instance, most subunits of V-type and F-type ATPases are co-regulated and cluster together during this phase. Interestingly, these types of ATPases exhibit different regulation patterns. Whereas F-type ATPase, also known as ATP synthase, is upregulated during diauxic shift, V-type ATPase is downregulated. ATPases are proton transporters using ATP in the process. In contrast to V-type ATPase, F-type ATPase (located in the inner membrane of mitochondria) is usually driven in reverse by chemiosmosis to produce ATP. This becomes increasingly necessary as the ATP supply through the glycolysis chain ceases. Other examples include the formation of cytochrome c oxidase which is upregulated to enhance the capability of yeast to produce ATP in the respiratory chain, and the upregulated succinate dehydrogenase which plays a role in the TCA cycle. The average size of clusters during this period, however, is small (2 objects per cluster). Since the focus of this invention is the identification of co-regulated pathways, i.e. clusters exceeding a certain size, further local maxima of the silhouette coefficient are inspected. For very late cutting points one finds an artificial increase of the average silhouette value. At this point, huge clusters have formed which do not exhibit either tight correlation of expression nor small network distance. However, unconnected small clusters are present which are still far apart from the main component, thus resulting in a high average silhouette value. More interestingly, Figure 4 shows an alternative cutting point in section B, which leads to a clustering with pathway-like clusters with an average size of 9 objects per cluster. Table 1 depicts the largest resulting clusters which exhibit relatively high co-regulation.

Table 1:

| This table shows the 10 largest clusters with gene expression distance smaller than 0.3 sorted by combined average distance. Main constituting KEGG pathways are listed for each cluster; single nodes may belong to pathways not listed here. Column *regulation* describes characteristic expression pattern during diauxic shift. The three following columns give the number of objects, distinct ORFs and distinct EC classifiers, respectively. The last two columns give the average value for expression and normalized network distance within the respective cluster. |
|---|

| Id | Pathways | regulation | # objects | # ORFs | # EC | $\delta_{norm,net}$ | $\delta_{exp}$ |
|---|---|---|---|---|---|---|---|
| A | Purin and Pyrimidine metabolism with complexes DNA / RNA polymerases, V-type ATPase; part of Aminosugar metabolism | down | 174 | 71 | 27 | 49.84 | 0.24 |
| B | Sterol biosynthesis and Glycoprotein biosynthesis; fragment of Fructose metabolism | down | 51 | 38 | 22 | 51.83 | 0.29 |
| C | Purin and Histidine metabolism; parts of Folate biosynthesis and Pyrimidine metabolism | down | 75 | 57 | 51 | 71.50 | 0.26 |
|  | TCA cycle and |  |  |  |  |  |  |
|  | Glutamate metabolism |  |  |  |  |  |  |
| E | Glycolysis, Pentose phosphate pathway; Starch metabolism; start of Phenylalanin, Tyrosin and Tryptophane metabolism | down | 83 | 50 | 38 | 70.82 | 0.29 |

Table 1: (continued)

| | Id | Pathways | regulation | # objects | # ORFs | # EC | $\delta_{norm,net}$ | $\delta_{exp}$ |
|---|---|---|---|---|---|---|---|---|
| This table shows the 10 largest clusters with gene expression distance smaller than 0.3 sorted by combined average distance. Main constituting KEGG pathways are listed for each cluster; single nodes may belong to pathways not listed here. Column *regulation* describes characteristic expression pattern during diauxic shift. The three following columns give the number of objects, distinct ORFs and distinct EC classifiers, respectively. The last two columns give the average value for expression and normalized network distance within the respective cluster. | | | | | | | | |
| | F | Phenylalanin, Tyrosin and Tryptophane metabolism; part of Folate biosynthesis | down | 41 | 25 | 22 | 74.43 | 0.25 |
| | G | Amino acid metabolisms: Valine, Leucine, Isoleucin metabolism; Glycine, Serine, Thrionine, Methionine metabolism; Selenoamino acid metabolism | down | 121 | 68 | 59 | 79.28 | 0.25 |
| | H | Purin metabolism with F-type ATP synthase | up | 52 | 33 | 19 | 70.86 | 0.28 |
| | I | Pyruvate metabolism; Selenoamino acid metabolism; Valine, Leucine, Isoleucine degradation | down | 39 | 18 | 17 | 83.83 | 0.28 |
| | J | Starch and Sucrose metabolism; Glycerolipid metabolism; part of Glycolysis, Fructose, Mammose and Galactose pathway | up | 94 | 60 | 51 | 79.85 | 0.29 |

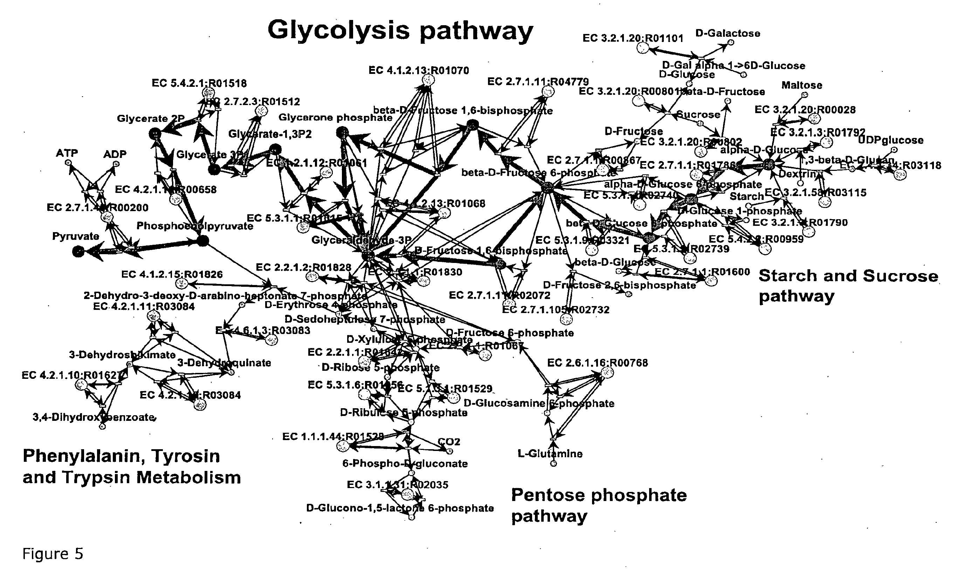

Fig. 5 shows the main part of Cluster E of Table 1. The cluster contains the down-regulated glycolysis pathway (marked with thick edges) and fragments of several directly connected reactions with co-regulated catalyzing enzymes. Some unconnected nodes have been omitted. Either, these are nodes with duplicate EC identifiers catalyzing different reactions, or nodes connected via few missing enzymes to the main component.

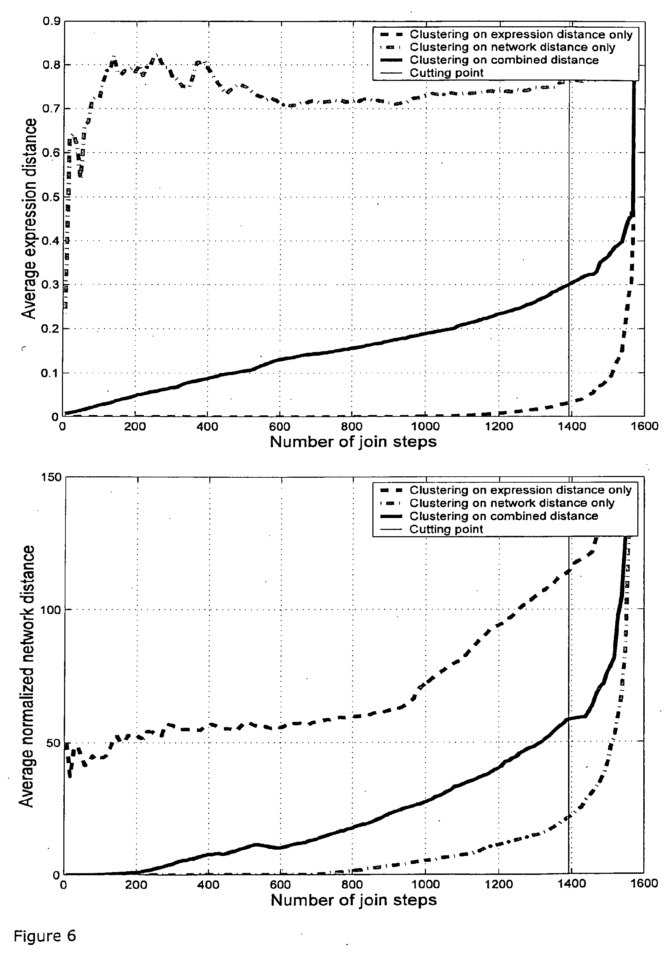

These clusters are able to paint a picture similar to the one extracted manually by DeRisi and co-workers. Their main observations are covered by clusters E (down-regulated glycolysis pathway), D (up-regulated TCA cycle) and J (up-regulated carbohydrate storage pathways). During growth in a sugar-rich medium, the yeast cell employs the glycolysis pathway for energy production. This pathway constitutes the main part of cluster *E,* which is illustrated in more detail in Figure 5. The expression pattern of cluster *E* shows down-regulation of genes during diauxic shift. This is due to the fact that the yeast cells turn to ethanol as alternative energy source, when the sugar in the medium is exhausted. This pathway is marked in Figure 5 with bold edges. Cluster E does not contain edges from the glycolysis pathway, only. Parts of the pentose phosphate pathway, which constitutes an alternative for conversion of glucose 6-phosphate into pyruvate for energy production, are included in the cluster. Moreover, reactions are attached which convert other types of sugar (e.g. sucrose or UDPglucose) to $\alpha$-D-Glucose. At the end of the glycolysis pathway, one finds reactions that channel phosphoenolpyruvate to the phenylalanin, tyrosin and tryptophane metabolism. All of these additional pathways also exhibit down-regulation during diauxic shift. In contrast, one finds up-regulation during diauxic shift for all genes in cluster *D*, which is mainly composed of the TCA cycle. This cycle is essential in aerobic growth, as it provides energy using acetyl-CoA as its source. In this cluster, one finds additional reactions from the glutamate metabolism. Here, 2-oxoglutarate is transaminated to 4-aminobutyrate which in turn can be transformed to succinate by succinsemialdehyde. This reaction chain avoids oxidative decarboxylation in favor of nitrogen-containing products. In cluster *A*, one finds DNA- and RNA-polymerases and the V-type ATPase, already discussed before, together with some supporting reactions from the purin and pyrimidine metabolism. This cluster shows consistent down-regulation. DNA- and RNA-polymerase activity as well as ATPase is reduced due to scant energy resources. Cluster *H*, in contrast, contains the F-type ATPase, parts of the purin metabolism and parts of the riboflavin metabolism. F-type ATPase is used for ATP production, and riboflavin metabolism may be activated to produce riboflavin and in turn FAD, which is used for energy production in the TCA cycle. This cluster contains genes enabling alternative ways of energy production in response to the ceasing supply of glucose. The final cluster *J* incorporates one key player responsible for the switch from glycolysis to gluconeogenesis (FBP1), together with pathways which support the channelling of glucose away to the carbohydrate storage pathways (e.g. starch metabolism). Again, this cluster corresponds well to a set of genes manually identified by DeRisi and co-workers to be involved in the described processes. It has to be noted that outliers, i.e. reactions that are not connected to the main component of a cluster, are usually present. This situation is to be expected as clustering is a heuristic procedure and the hierarchical clustering algorithm employed here is susceptible to noise-induced instability. The big picture of the clusters presented here was stable against changes of the parameters. This leads to the conclusion that resampling or bootstrapping methods should be applied to detect the reliable cores of the computed clusters. Nonetheless, the generated clustering, as shown above, helps to quickly obtain a picture of metabolic changes indicated by the gene expression data. In contrast to the results shown above, clusters are evaluated based on expression or network distances only. Figure 6 shows a comparison of clustering based on three distance functions: $\delta_{exp}$ (expression only), $\delta_{norm,net}$ (normalized network only) and $\Delta_{norm}$(combined).

Fig. 6 shows a comparison of clustering based on three distance functions: expression distances only, normalized network distances only and normalized combined distances. Upper figure shows average intra-cluster *expression distance,* lower figure shows average intra-cluster *normalized network distance.*

For every feasible cutting point of the resulting dendrograms, one evaluates the average distance of each object to all objects within the same cluster. The average of this value over all objects provides a measure for the compactness of a clustering according to a specific distance function. One computes this value separately for all three distance functions. Figure 6 indicates that clustering based on network distance or expression distance alone is not sufficient to arrive at co-regulated pathway-like clusters. When gene expression distance is clustered, the corresponding network distance is high and vice versa. This means that one would either end up with sets of well co-regulated genes which are scattered over the network or with a compact part of the network which is not co-regulated. The combined distance function, however, is able to yield clusters with low average distance according to network and expression distance function simultaneously, which shows that the method successively incorporated joint information on regulation and network proximity into the clustering process.

**Advantages of the Invention**

In comparison to (gene) expression clustering as described in the literature, the invention makes use of knowledge of biological networks. In comparison to mapping (gene) expression clusters onto biological networks as described in the literature, the invention incorporates knowledge of biological networks earlier. This can prevent the generation of wrong clusters. Late integration as proposed in the literature may make it necessary to split clusters that cover several pathways and, at the same time, to select several clusters to be able to cover a single pathway. Examples can

EP 1 318 472 A2

be found in [4].

In comparison to pathway scoring or similar methods as described in the literature, the invention

- is computationally more efficient, since the combinatorial explosion arising from the computation of pathways from the network is avoided;

- is more general, since it does not rely on the existance of a notion of pathways, and can thus be applied to any kind of molecular biological graph;

- avoids ambiguities that may arise from pathways that differ in details and achieve similar scores.

The invention differs from the method described by Ogata et al. in [10] in several respects:

In the invention, the two data types are represented by distance matrices, while the approach of Ogata et al. is based on the concept of graphs' [10]. Graphs are discrete structures that require simplification of the data. They can be seen as very basic distance functions that allow for only two different distances.

Ogata et al. extend a simple existing graph clustering procedure to simultaneously use two graphs. This can be difficult for more complex clustering algorithms. In contrast, in the described invention, the two data structures are first merged and then clustered. This allows for the use of available clustering algorithms without modification.

The goal of the invention is the determination of biologically reasonable pathways. In contrast, Ogata et al. describe 'a simple method for graph comparison' [10].

<u>References</u>

1. Y. Cheng and G. M. Church.
Biclustering of expression data.
In *Proceedings of the 8th International Conference on Intelligent Systems for Molecular Biology*, volume 8, pages 93-103, 2000.

2. DeRisi, J. L., Iyer, V. R. & Brown, P. O. (1997).
Exploring the metabolic and genetic control of gene expression on a genomic scale.
*Science,* **278**, 680-685.

3. Michael B. Eisen, Paul T. Spellman, Patrick O. Brown, and David Botstein. Cluster analysis and display of genome-wide expression patterns.
*Proceedings of the National Academy of Sciences of the USA*, 95(25): 14863-14868, December 1998.
Genetics.

4. M. Fellenberg and H. Werner Mewes.
Interpreting Clusters of Gene Expression Profiles in Terms of Metabolic Pathways.
In *Proceedings of the German Conference on Bioinformatics '99,* 1999.
Poster.

5. A. J. Hartemink, D. K. Gifford, T. S. Jaakkola, and R. A. Young.
Using Graphical Models and Genomic Expression Data to Statistically Validate Models of Genetic Regulatory Networks.
In *Proceedings of the Pacific Symposium on Biocomputing '01*, pages 422-433, 2001.

6. Trevor Hastie, Robert Tibshirani, Michael B Eisen, Ash Alizadeh, Ronald Levy, Louis Staudt, Wong C Chan, David Botstein, and Patrick Brown.
'Gene shaving' as a method for identifying distinct sets of genes with similar expression patterns.
*Genome Biology,* August 2000.

7. K. Jenssen, A. Laegreid, J. Komorowski, and Hovig E.
A literature network of human genes for high-throughput analysis of gene expression. *Nature Genetics,* 28(1): 21-28, May 2001.

8. Robert Küffner, Ralf Zimmer, and Thomas Lengauer.
Pathway analysis in metabolic databases via differential metabolic display (DMD).
*Bioinformatics,* 16(9):825-836, 2000.

9. Mitsuteru Nakao, Hidemasa Bono, Shuichi Kawashima, Tomomi Kamiya, Kazushige Sato, Susumu Goto, and Minoru Kanehisa.
Genome-scale Gene Expression Analysis and Pathway Reconstruction in KEGG.
In Asai, K., Miyano, S., Takagi, and T., editors, *Genome Informatics,*
volume 10, pages 94-103, Tokyo. Universal Academy Press.

10. H. Ogata, W. Fujibuchi, S. Goto, M. Kanehisa .
A heuristic graph comparison algorithm and its application to detect functionally related enzyme clusters.
*Nucleic Acids Research,* 28(20):4021-4028, 2000.

11. Eran Segal, Ben Taskar, Audrey Gasch, Nir Friedman, and Daphne Koller. Rich probabilistic models for gene expression.
*Bioinformatics,* 17(Supplement 1):S243-S252, June 2001.

12. R. Sharan and R. Shamir.
CLICK: a clustering algorithm with applications to gene expression analysis.
In *Proceedings of the 8th International Conference on Intelligent Systems for Molecular Biology,* volume 8, pages 307-316, 2000.

13. Amos Tanay and Ron Shamir.
Computational expansion of genetic networks.
*Bioinformatics,* 17(Supplement 1):S270-S278, June 2001.

14. J. van Helden, D. Gilbert, L. Wernisch, K. Schroeder, and S. Wodak.
Applications of regulatory sequence analysis and metabolic network analysis to the interpretation of gene expression data.
*Lecture Notes in Computer Sciences,* 2000.

15. Alexander Zien, Robert Küffner, Ralf Zimmer, and Thomas Lengauer.
Analysis of Gene Expression Data with Pathway Scores.
In Russ Altman et al., editors, *Proceedings of the 8th International Conference on Intelligent Systems for Molecular Biology,* pages 407-417, La Jolla, CA, August 2000. AAAI.

**Claims**

1. A method for the joint analysis of molecular expression data and biological networks by clustering comprising the steps of

   - defining a matrix of distances between molecules or sets of molecules that incorporate both the relation of corresponding expression profiles and information on their relation within a biological network; and

   - clustering the molecules based on said distances.

2. A method for the joint analysis of molecular expression data and biological networks as in claim 1, where the distance between two molecules is defined by combining a distance function on the corresponding expression data with a distance function on the biological network, such that the resulting distance of two molecules is monotonically increasing with increasing expression data distance for constant biological network distance, and monotonically increasing with increasing biological network distance for constant expression data distance.

3. A method for the joint analysis of molecular expression data and biological networks as in claim 2, where the distance between two molecules is defined as infinite (or prohibitively high) if

   - said molecules are not adjacent in a given biological network;

- said molecules are nodes in a given biological network but are not adjacent to each other;

- said molecules are not closer to each other (in terms of path length) than a given threshold in a given biological network; or

- said molecules are more distant to each other (in terms of path length) than a given threshold in a given biological network;

and where the distance is calculated from the expression data alone, otherwise.

4. A method for the joint analysis of molecular expression data and biological networks as in claim 2, where the distance between two molecules is defined as infinite (or prohibitively high) if the corresponding expression distance exceeds a given threshold, and where the distance is calculated from the biological network alone otherwise.

5. A method for the joint analysis of molecular expression data and biological networks as in claim 1, where the distance between two molecules is defined by combining a score deduced from the expression data with a distance function deduced from the biological network, where the biological network distance may be either continuous or discrete.

6. A method for the joint analysis of molecular expression data and biological networks as in claim 5, where the score of a molecule reflects the degree of differential expression between two states or types of cells.

7. A method for the joint analysis of molecular expression data and biological networks as in claim 1, where the distance between two molecules is defined by combining a score deduced from the biological network with a distance function deduced from the expression data, where the expression distance may be either continuous or discrete.

8. A method for the joint analysis of molecular expression data and biological networks as in claim 1 to claim 7, where the clustering algorithm is

- an average linkage clustering algorithm;

- a hierarchical clustering algorithm;

- k-means clustering;

- self-organizing networks (SOMs); or

- a centroidal clustering algorithm

- a hypergraph clustering algorithm.

9. A method for the joint analysis of molecular expression data and biological networks as in claim 1 to claim 8, where the molecular expression data is

- gene expression data;

- protein expression data; or

- metabolite abundances.

Figure 1

Figure 2

Figure 3

Figure 4

Figure 5

EP 1 318 472 A2

Figure 6